(19) Europäisches Patentamt
European Patent Office
Office européen des brevets

(11) EP 0 957 075 A1

(12) **EUROPÄISCHE PATENTANMELDUNG**

(43) Veröffentlichungstag:
**17.11.1999 Patentblatt 1999/46**

(51) Int. Cl.[6]: **C07C 45/67**, C07C 45/82, C07C 49/603

(21) Anmeldenummer: **99108497.1**

(22) Anmeldetag: **30.04.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **13.05.1998 DE 19821379**

(71) Anmelder:
**Degussa-Hüls Aktiengesellschaft
60287 Frankfurt am Main (DE)**

(72) Erfinder:
- **Krill, Steffen Dr.
67346 Speyer (DE)**
- **Kretz, Stephan
63599 Biebergemünd (DE)**
- **Hasselbach, Hans-Joachim Dr.
63571 Gelnhausen (DE)**
- **Huthmacher, Klaus Dr.
63584 Gelnhausen (DE)**
- **Hahn, Rainer Dr.
63654 Büdingen (DE)**
- **Schmitt, Hermann
63517 Rodenbach (DE)**

(54) **Verbessertes Verfahren zur Herstellung von 3,5,5-Trimethylcyclohexa-3-en-1-on (Beta-Isophoron) durch Isomerisierung von 3,5,5-Trimethylcyclohexa-2-en-1-on (Alpha-Isophoron) (II)**

(57) Verfahren zur Herstellung von 3,5,-Trimethylcyclohexa-3-en-1-on (β-Isophoron).

Die Erfindung betrifft ein Verfahren zur Herstellung der Titelverbindung durch Isomerisierung von 3,5,5-Trimethylcyclohexa-2-en-1-on (α-Isophoron) in der Flüssigphase in Gegenwart eines salzartigen oder metallorganischen Katalysators.

Durch die Verwendung katalytischer Mengen von Alkali- oder Erdalkalisalzen und und deren metallorganischen Verbindungen und ohne Zusatz eines weiteren Hilfsstoffes gelingt es, mit einem preiswerten, allgemein zugänglichen Katalysator die Raum-Zeit-Ausbeute bei der Herstellung von β-Isophoron auf ein für eine industrielle Anwendung geeignetes Niveau zu heben und gleichzeitig einen an sich bekannten Prozeß deutlich zu verbessern.

β-Isophoron ist besonders als Zwischenprodukt zur Herstellung von Ketoisophoron geeignet.

EP 0 957 075 A1

## Beschreibung

Die Erfindung betrifft ein Verfahren zur Herstellung von 3,5,5-Trimethylcyclohexa-3-en-1-on (β-Isophoron) durch Isomerisierung von 3,5,5-Trimethylcyclohexa-2-en-1-on (α-Isophoron) in der Flüssigphase in Gegenwart eines salzartigen Katalysators, der ein anorganisches Kation enthält.

β-Isophoron hat großes wirtschaftliches Interesse, da es einen wichtigen Synthesebaustein für die Herstellung von Carotinoiden, Vitaminen und pharmazeutischen Produkten darstellt. Insbesondere wird β-Isophoron als Vorstufe für Ketoisophoron (= 2,6,6-Trimethylcyclohex-2-en-1,4-dion) und Trimethylhydrochinon und damit für die Herstellung von Vitamin E benötigt. Daneben geht es zentral in Synthesen für Riechstoffe und Naturstoffe wie Astaxanthin und Abscisinsäure und Derivate ein.

Die Herstellung von Isophoron gelingt durch Aceton-Trimerisierung, unter Kondensation der $C_3$-Bausteine. Das hauptsächlich gebildete Isomere ist das α-Isophoron, da es im Gegensatz zum β-Isomeren eine zur Ketofunktion konjugierte Doppelbindung besitzt. Aus diesem Grund liegt das thermodynamische Gleichgewicht auf Seiten des α-Isophorons; die β-Konzentration beträgt nur etwa 1 - 2 % in Abhängigkeit der Temperatur und die Gleichgewichtseinstellung erfolgt sehr langsam.

Obwohl es grundsätzlich zwei verschiedenen Ansätze gibt, zum Ketoisophoron zu gelangen, nämlich die Direktoxidation von α-Isophoron → Ketoisophoron und dem Umweg über die Isomerisierung α-Isophoron → β-Isophoron in einem Primärschritt und anschließende Oxidation des β-Isophoron → Ketoisophoron, ist letzteres Verfahren deutlich vorteilig. Schema 1 stellt diese Überlegungen zur Ketoisophoron-Synthese anschaulich dar:

Schema 1: Allgemeines Syntheseprinzip zum Aufbau von KIP (= Ketoisophoron = 2,6,6-Trimethyl-2-cyclohexen-1,4-dion)

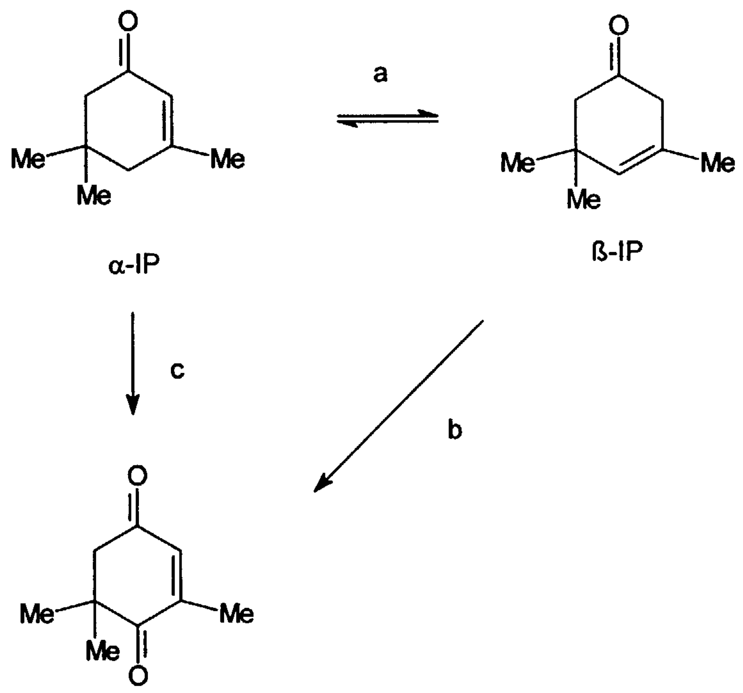


α-IP

ß-IP

KIP

a = Isomerisierung von α-IP zu β-IP

b = Oxidation von β-IP zu KIP

c = Direktoxidation α-IP zu KIP

Im Laufe der Zeit sind zahlreiche Verfahren zur Isomerisierung von α-IP beschrieben worden, die jedoch erhebliche Nachteile aufweisen. Gesichtspunkte wie hoher Chemikalienverbrauch, schlechte Raum-/Zeitausbeute und Schwierigkeiten bei der Aufarbeitung haben bisher eine praktische Verfahrensumsetzung im größeren Maßstab verhindert.

Bei den Herstellungsverfahren für β-IP aus α-IP kann man zwischen Gasphasen- und Flüssigphasen-Reaktionen unterscheiden.

Grundsätzlich sind vier parallele Reaktionen von α-Isophoron in der Gasphase möglich, die miteinander in Konkurrenz stehen und abhängig vom gewählten Temperaturbereich bzw. der Oberfächenbeschaffenheit des eingesetzten Katalysators in unterschiedlichem Ausmaß zum Zuge kommen.

Isophoron kann auf folgende Weise am Kontakt in der Gasphase reagieren:

a.) Isomerisierung zu β-Isophoron

b.) Reduktion zu Trimethylcyclohexadienen (hierzu benötigter Wasserstoff wird durch IP-Zersetzung geliefert, die von Verkokungserscheinungen begleitet wird)

c.) β-Eliminierung von Methan zu 3,5-Xylenol

d.) Mesitylen-Entstehung

Im folgenden Schema 2 sind die am heterogenen Kontakt in der Gasphase katalysierten Reaktionen von α-IP dargestellt:

Schema 2: Reaktivität von α-IP in der heterogenen Gasphasenkatalyse

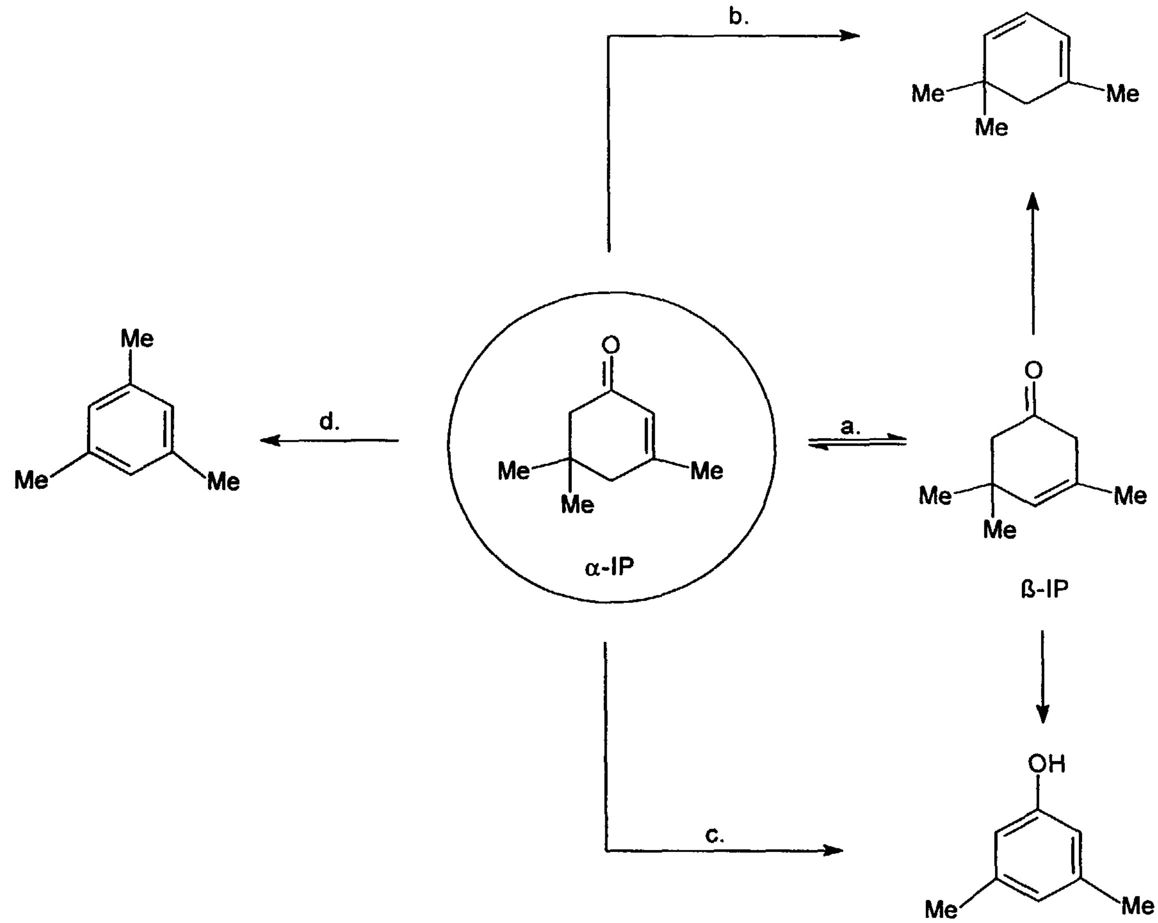


α-IP

ß-IP

Die EP 0 488 045 B1 offenbart ein Isomerisierungsverfahren in der Gasphase (300 - 450 °C) über einem heterogenen Katalysator. Als Katalysatoren werden Oxide und Mischoxide von Mg (Gr. IIa), Al (IIIa), Si (IVa) und Ni (VIII) verwendet, die per se aktiv sind oder auf einem γ-Aluminiumoxid-Träger aufgebracht werden (spezifische Oberfläche 1 - 50 $m^2/g$). Pro Liter Katalysator werden 1 - 10 kg α-IP eingesetzt, die Konzentration der intermediär erhaltenen Lösung beträgt je nach Katalysatorbelastung maximal 9 % β-IP, die Endkonzentration nach Destillation im Vakuum 97 % β-IP. Die Granulierung von NiO erfolgt mit 1 % Luviskol K90 (-Polyvinylpyrrolidon). Unter optimalen Bedingungen wird bei der Anwendung dieser Verfahrensweise eine Katalysatorleistung von 0,33 l $\beta\text{-IP/h/l}_{Kat.}$ erreicht.Bezogen auf das eingesetzte Eduktvolumen beträgt die Raum-Zeit Ausbeute $Y_{R\text{-}Z}$ = 0,09 $l_{\beta\text{-IP}}/h/l_{Lösung}$ (Bsp.1)

Zudem ist die Abnahmegeschwindigkeit gering, was das Verfahren im industriellen Maßstab wenig attraktiv macht.

Bei L. *F. Korzhova, Y. V. Churkin und K. M. Vaisberg, Petrol. Chem Vol. 31, 1991, 678* wird die Umsetzung von α-IP bei 300 - 800 °C in Gegenwart heterogener Katalysatoren beschrieben. Als katalytische Systeme werden γ-Aluminiumoxid, Magnesiumoxid und Quarz berücksichtigt. Das Produktspektrum wird in Abhängigkeit von Temperatur und Katalysator betrachtet. Verglichen miteinander werden die Bildung von β-IP, Trimethylcyclohexadien, 3,5-Xylenol, und Mesitylen (siehe Schema 2: Weg a., b., c.; d.). So ergibt die thermische Umsetzung von α-IP bei über 550 °C an einer wenig ausgebildeten katalytischen Oberfläche (Quarz) ein Gemisch der Zusammensetzung c >> a >> d und b = 0. Die Umsetzung am MgO-Kontakt bei 400 °C zeigt ein ähnliches Produktbild bei deutlich niedrigerer Temperatur, näm-

lich c >> a > d > b . In Gegenwart eines Aluminiumoxidkatalysators mit ausgeprägt basisch-saurer Oberflächenstruktur erfolgt die Umsetzung bei 300 °C mit deutlicher Bevorzugung der Cyclohexadien Produkte, nämlich b >> c > d .

Insgesamt ist davon auszugehen, daß eine katalytische Gasphasenisomerisierung durchaus auf mehrfache Weise nachteilig ist: Allgemein läßt sich sagen, daß diese Verfahren sich nachteilig darstellen, weil entweder die Produktbildung von einem erheblichen Nebenproduktanfall begleitet wird, oder die Raum-Zeit-Ausbeute (absolute β-IP-Entstehung/h/$kg_{Kat}$) zu gering ist.

Auch mit der Isomerisierung in der Flüssigphase beschäftigen sich eine Reihe von Druckschriften. Der nähere Stand der Technik wird durch folgende Schriften repräsentiert:

D1 = A. Heymes et al., Recherches 1971, 18, 104
D2 = FR-A-1 446 246
D3 = DE-OS-24 57 157
D4 = US-A-4,005,145
D5 = EP-A-0 312 735
D6 = JP 87-33019 entspr. HEI-1-175954 v. 12.07.1989

D1 offenbart die Isomerisierung von α-IP zu β-IP mit stöchiometrischen Mengen MeMgX (X = Halogen-) Grignard Verbindung. Man erhält unter Methanfreisetzung in Gegenwart katalytischer Mengen $FeCl_3$ 73 % β-IP. Mechanistische Vorstellungen gehen davon aus, daß die Grignardverbindung als Base reagiert und nicht als Überträger eines Carbanions fungiert. Überschüssiges Mg führt zur Entstehung von Dimerengemischen, die aus einer reduktiven metallischen Dimerisierung hervorgehen. Die Umsetzung von α-Isophoron mit molaren Mengen Methylmagnesiumjodid in Gegenwart katalytischer Mengen $FeCl_3$, anschließende Hydrolyse und destillative Aufarbeitung ist jedoch ebenso kompliziert wie chemikalienaufwendig.

D2 betrifft die Isomerisierung von α-IP zu β-IP in Gegenwart katalytischer Mengen p-Toluolsulfonsäure und allgemein aromatischer Sulfonsäuren, insbesondere Anilinsulfonsäure. Die Einsatzmenge des Katalysators beträgt bezogen auf eingesetztes α-IP 0,1 - 0,2 %. Ein niedriger Umwandlungsgrad und ein hoher Nebenproduktanfall verhindern jedoch eine industrielle Anwendung des Verfahrens der D2.

Nach der D3 erfolgt die Darstellung von β-IP durch mehrstündiges Kochen von α-IP in Triäthanolamin, Fraktionierung, Waschen des Destillates mit Weinsäure und Kochsalzlösung. Auch hier ist der Chemikalienverbrauch und der Verfahrensaufwand erheblich.

In der D4 werden als Katalysator Säuren eingesetzt mit einem pK = 2 - 5 und einem höheren Siedepunkt als β-IP (Sdpkt. β-IP = 186 °C/760 mm Hg). Gemäß Patentanspruch sind in der flüssigen Phase explizit geschützt:

Aliphatische und aromatische Aminosäuren, Adipinsäure, p-Methyl-Benzoesäure, 4-Nitro-m-Methyl-Benzoesäure, 4-Hydroxy-Benzoesäure, 3,4,5-Trimethoxybenzoesäure, Vanillinsäure, 4-Trifluormethyl-Benzoesäure, 3-Hydroxy-4-Nitrobenzoesäure und Cyclohexancarbonsäure und Derivate.

Der Katalysatoreinsatz beträgt 0,1 - 20 Mol %. Die Ausbeute an β-IP (bezogen auf eingesetztes α-IP) und damit die Sektivität beträgt 74,5 %. Dies entspricht unter den gegebenen Bedingungen umgerechnet auf die eingesetzte Katalysatormenge und Zeit einer Ausbeute von Y = 0,218 Liter β-IP pro Kilogramm Katalysator und Stunde.

Die homogen-katalytische Isomerisierung von α-IP→β-IP mit wenig dissoziierten Säuren stellt eine Verbesserung bezüglich des Chemikalienverbrauchs dar, wobei β-IP kontinuierlich aus dem Gleichgewicht entfernt wird. Mit einer so geringen Abnahmegeschwindigkeit wie 11 ml/h β-IP bei einer Einsatzmenge von etwa 0,5 kg α-IP ist die Raum-Zeit-Ausbeute und die β-IP-Entstehung mit Y = 0,24 kg β-IP/$kg_{Kat}$/h zu niedrig, um technische Anwendung zu finden.

Nach einem ähnlichen Prinzip geht man in D5 vor. Als π-Bindungsverschiebungskatalysatoren finden Acetylacetonate von Übergangsmetallen Verwendung. Auch $Al(acac)_3$ zeigt katalytische Aktivität. Der Einsatz des Katalysators erfolgt in 0,01 - 10 wt %. Patentiert sind Metallkatalysatoren der Gruppen IVb (Ti/Zr/Hf), Vb (V/Nb/Ta), VIb (Cr, Mo, W), VIIb (Mn/ Tc/Re) der gesamten Gruppe VIII und Aluminium. Das primär anfallende Destillat hat einen β-IP-Gehalt von 94 %, eine weitere Vigreuxdestillation reichert den β-IP-Gehalt auf 99 % an. Dieses Ergebnis entspricht bezogen auf Katalysatoreinsatzmenge und Zeit einer Ausbeute von Y = 9,4 Liter β-IP pro Kilogramm Katalysator und Stunde.Bezogen auf die eingesetzte Eduktlösung entspricht dies einer Ausbeute von $Y_{R-Z}$ = 0,0376 $l_{\beta\text{-IP}}$/h/$l_{Lösung}$.

Abgesehen davon, daß die Raum-Zeit-Ausbeute gering und der Nebenproduktanfall beträchtlich ist, lassen sich bei dem eingesetzten homogenen Katalysatorsystem Katalysator und Destillationsrückstand nicht ohne weiteres trennen. Daher ist ein Verwerfen von Zeit zu Zeit unerläßlich, da die Temperatur im Destillationssumpf sonst zu sehr ansteigen würde. Auch so ist schon ein "Nachziehen" der Temperatur erforderlich.

Gemäß D6 erfolgt die Isomerisierung in der Flüssigphase bei Temperaturen um 200 °C. Als Katalysator finden Kieselgel mit oder ohne Zusatz von alkylsubstituierten Imidazolinen der nachfolgenden Formel Verwendung.

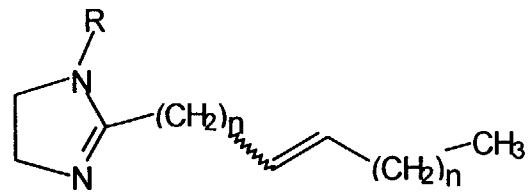

Typische Experimentbedingungen: 300 g α-IP und 25,7 g $SiO_2$ werden 52 h in Gegenwart von Edelstahl destilliert, es resultieren 230 g β-IP (= 76,6 % Ausbeute) mit 99,9 % Reinheit. Dieses Ergebnis entspricht bezogen auf Katalysatoreinsatzmenge und Zeit einer Ausbeute von Y = 0,174 Liter β-IP pro Liter Katalysator und Stunde. Die Präparation der organischen Basen ist jedoch teuer und die Raum-Zeit-Ausbeute des Verfahrens gering; mit einem charakteristischen Wert von Y = 0,174 Liter β-IP/l Kat/h ist auch dieses Verfahren nicht in einen technischen Maßstab umzusetzen.Bezogen auf das Volumen der eingesetzten Eduktlösung beträgt die Ausbeute $Y_{R\text{-}Z}$ = 0,0149 $l_{\beta\text{-}IP}/h/l_{Lösung}$.

Die beschriebene Verfahrensweise ist zudem ungünstig, die absolute β-IP-Entstehung gering. Besonders nachteilig ist die batchweise Durchführung und die Durchführung von Isomerisierung und Reindestillation des β-IP in einem Schritt. Durch die hohe Reaktionstemperatur in der Destillationsapparatur tritt nachweislich im erheblichen Maße die Rückisomerisierung von β-IP zu α-IP ein.

Angesichts des hierin zitierten und diskutierten Standes der Technik war es eine Aufgabe der Erfindung, die oben erwähnten Nachteile der bisherigen Methoden zu vermeiden und ein Verfahren anzubieten, nach dem auf technisch vorteilhafte Weise 3,5,5-Trimethylcyclohexa-3-en-1-on aus seinem Isomeren 3,5,5-Trimethylcyclohexa-2-en-1-on hergestellt werden kann. Insbesondere war das Auffinden eines verbesserten katalytischen Verfahrens in der Flüssigphase bei Reduzierung der bisher hohen Katalysatoraufwandmengen ein besonderes Ziel der Erfindung.

Gelöst werden diese sowie weitere nicht einzeln angegebene Aufgaben bei einem Verfahren der eingangs genannten Gattung durch die im kennzeichnenden Teil des Anspruches 1 und den folgenden Ansprüchen angegebenen Maßnahmen, ohne daß weitere organische Basen zugesetzt werden müssen.

Als salzartige Katalysatoren werden insbesondere Alkalioxide, Alkali- und Erdalkali Hydroxide, Hydrogencarbonate, Carbonate, Cyanide, Fluoride, Bromide, Alkoholate, Sulfide, Hydrogensulfide, Hydrogensulfate, Mercaptide, Enolate, Carboxylate, Hydride, komplexen Hydride, Amide oder entsprechende Alkali- und Erdalkaliverbindungen, die als Gegenion ein Carbanion enthalten, oder direkt die metallischen Elemente verwendet. Die genannten Verbindungen setzt man gegebenfalls auch in Form von Lösungen zu, wobei als Lösungsmittel sowohl Wasser als auch organische Verbindungen geeignet sind. Bevorzugt werden die Alkaliverbindungen verwendet.

Erfindungsgemäß gelingt es, die Raum-Zeit-Ausbeute bei der Herstellung von β-Isophoron durch Isomerisierung von α-Isophoron deutlich zu steigern, die Katalysatoraufwandmengen drastisch zu reduzieren und gleichzeitig einen im Prinzip bekannten Prozeß wesentlich zu vereinfachen.

Das erfindungsgemäße Verfahren ermöglicht einen hohen Umsatz im Bereich von ca. 422,15 kg β-Isophoron pro kg eingesetztem Katalysator und pro Stunde (exemplarische Kalkulation: 0,2 mmol $K_2CO_3$ = 27,642 mg für 1 Mol alpha-IP = 138,21 g/Mol: d.h. 200,00 mg Kaliumcarbonat für 1 kg IP bei 74 g β-IP/h/l = 80,43 g β-IP/h/kg Lösung)und übertrifft dadurch die bislang im Stand der Technik bekannten Verfahren bei weitem. Weiterhin werden erfindungsgemäß sowohl der Anfall von Nebenprodukten verringert als auch die Raum-Zeit-Ausbeute bezogen auf das Volumen der eingesetzten Eduktlösung mit $Y_{R\text{-}Z}$ = 80 g β-IP/h pro kg α-IP verbessert. Insgesamt gesehen, ist die Benutzung der erfindungsgemäßen Salze und metallorganischen Verbindungen als Katalysatoren in jedem Falle vorteilhaft.

In der vorliegenden Erfindung wird ein Verfahren eingesetzt, in dem α-Isophoron zu seinem Isomeren β-Isophoron umgesetzt wird, wobei insbesondere alkalische Salze und metallorganische Verbindungen als Katalysatoren in der Flüssigphase verwendet werden.

Besonders vorteilhaft ist eine Verfahrensweise, bei der Reaktion und Produktisolierung nicht in derselben Apparatur stattfinden. Dadurch, daß man in einer Isomerisierungseinheit zunächst ein Gemisch von α-IP und β-IP erzeugt, und dann die Reindestillation im Vakuum durchführt, kann die Raum-Zeit-Ausbeute deutlich gesteigert werden, da bei einer Aufkonzentration von β-IP bei Normaldruck und der Siedetemperatur von 186° C teilweise Rückisomerisierung und Nebenproduktbildung eintritt, die durch schnelles Entfernen der Reaktionsmischung aus dem Reaktionsraum verhindert wird. Im einfachsten Fall legt man die Mischung bestehend aus dem salzartigen Katalysator und α-IP vor und thermolysiert diese Mischung in einem Reaktionsrohr oder Reaktor bei einer geeigneten Temperatur. Das Reaktionsprodukt enthält gemäß der eingestellten Verweilzeit und Temperatur die der Gleichgewichtskonzentration entsprechenden Mischung aus α-IP und β-IP. Das so gebildete β-Isophoron wird destillativ abgetrennt, nicht umgesetztes α-Isophoron wird zusammen mit dem Katalysator unter Ergänzung von Frisch-α-IP dem Reaktionsrohr oder Reaktor wieder zugeführt.

In einem anderen dem erfindungsgemäßen Verfahren entsprechenden Vorgehen legt man die Mischung Katalysator/α-IP in einem Reaktor vor und nimmt über eine Destillationskolonne ein Kopfprodukt der Zusammensetzung von bis zu 50 wt% β-IP in α-IP ab, das anschließend in einer weiteren Kolonne so aufgetrennt wird, daß als Kopfprodukt reines β-IP abgenommen wird. Das nicht umgesetzte α-IP wird erneut dem Isomerisierungsreaktor zugeführt.

Als Katalysatoren im Sinne der Erfindung werden die genannten Salze eines Elements der Gruppen $I_a$ und $II_a$ des Periodensystems eingesetzt. Die Gruppeneinteilung der Haupt- und Nebengruppen des Periodensystems der Elemente erfolgt nach der Bezeichnung gemäß *IUPAC, Pure and Appl. Chem., 66, 2423-2444,1994.* So gehören zur Gruppe $I_a$ die Metalle Li, Na, K, Rb, Cs und zur Gruppe IIa die Elemente Be, Mg, Ca, Sr, Ba.

Zu den erfindungsgemäß als salzartige Katalysatoren einsetzbaren Verbindungen gehören die Oxide der Gruppe Ia, und die Hydroxide, Hydrogencarbonate, Carbonate, Fluoride, Bromide, Alkoholate, Sulfide, Hydrogensulfide,Hydrogensulfate, Mercaptide, Enolate, Carboxylate, Hydride, komplexen Hydride, Amide der Elemente der Gruppen Ia und IIa oder entsprechende Alkali- und Erdalkaliverbindungen, die als Gegenion ein Carbanion enthalten, die metallischen Elemente und sonstige katalytisch aktiven Alkali- und Erdalkalisalze der Gruppen Ia und IIa, die als Gegenion ein ein - oder mehrfach negativ geladenes Anion besitzen. Unter diesen Gegenionen sind unter anderem auch Cyanide, Halogenide (ohne Chloride), Hydrogensulfate katalytisch aktiv. Auch die metallischen Elementverbindungen lassen sich als Katalysatorquelle benutzen, die in situ mit α-IP und im α-IP enthaltenen Nebenprodukten die katalytisch aktive Spezies bilden. Unter metallorganischen Basen sollen diejenigen Verbindungen der Alkali- und Erdalkalimetalle erfasst sein, in denen ein Carbanion das Gegenion darstellt. Zu den im Rahmen der Erfindung einsetzbaren Carbanionen gehören $sp^3$-, $sp^2$-, und sp- hybridisierte Carbanionen. Ohne Anspruch auf Vollständigkeit zu erheben seien als Katalysatorquelle der Isomerisierung die folgenden Verbindungen genannt: Alkali- und Erdalkali Methyl-, Ethyl, sec-Propyl, Propyl, n-Butyl, Phenyl-, Tolyl-, Benzyl-, Cyclopentadienyl-Verbindungen. Als Vertreter der $sp^2$- und $sp^3$ hybridisierten Carbanionen seien Vinyllithium und Natriumacetylid genannt. Unter dem Terminus Amide versteht man die Metall-Salze der erfindungsgemäßen Metalle mit primären und sekundären Aminen. Als Vertreter dieser Verbindungsklasse seien hier Natriumamid und Natriumsuccinimid angeführt.

Es ist ebenso Gegenstand der vorliegenden Erfindung, daß auch andere, im beschriebenen Konzentrationsbereich und unter den vorgegebenen Bedingungen basisch wirkende, metallorganische Verbindungen genannter Elemente mit einem $pK_B$-Wert >5 als Katalysatoren der Umsetzung eingesetzt werden können.

Zu den im Rahmen der Erfindung einsetzbaren Alkoholate und Mercaptide zählen neben den Verbindungen, die sich von den gebräuchlichen aliphatischen Alkoholen und Mercaptanen wie Methanol, Ethanol oder tert-Butanol und anderen ableiten auch die Phenolate und davon abgeleitete Derivate.

Von den obengenannten Katalysatoren sind insbesondere diejenigen bevorzugt, welche ein Element der Gruppen $I_a$ des Periodensystems enthalten.

Ganz besonders bevorzugt werden im Rahmen der Erfindung die Carbonate und Hydroxide des Lithiums, Natriums oder des Kaliums verwendet.

In einer weiteren bevorzugten Ausgestaltung des erfindungsgemäßen Verfahrens werden bevorzugt die Alkalimetalle und Erdalkalimetalle als Elemente eingesetzt.

Besonders bevorzugt bei dieser Variante ist die Benutzung von metallischem Lithium, Natrium und Kalium.

Ganz besonders bevorzugte Basen sind u. a. Natriumcarbonat oder Kaliumcarbonat oder die entsprechenden Hydroxidverbindungen.

Die Menge des eingesetzten Katalysators ist an sich nicht besonders kritisch und kann daher über einen weiten Bereich variiert werden. Bevorzugt ist es, daß die Base in einer Menge von 0,0001 bis 5 Mol-% (Mol/Mol) bezogen auf das vorgelegte α-Isophoron eingesetzt wird.

In einer besonders bevorzugten Ausführungsvariante kennzeichnet sich das Verfahren der Erfindung dadurch, daß der Katalysator in einem Verhältnis von zwischen 0,001 und 5 Mol-% (Mol/Mol) bezogen auf α-Isophoron eingesetzt wird.

In noch einer weiteren besonders bevorzugten Ausführungsform liegt das Verhältnis von Katalysator zu α - Isophoron im Bereich zwischen 0,01 - 2 Mol-%.

Das Verfahren gemäß Erfindung wird in einem Temperaturbereich zwischen 100 und 400 °C ausgeführt. Bevorzugt ist der Temperaturbereich zwischen 100 und 260 °C, insbesondere 150 bis 250 °C.

Der Zusatz eines Verdünnungs- oder Lösungsmittels ist zwar möglich, aber nicht erforderlich.

Vorzugsweise wird die Reaktion bei einem Druck von 10 mbar bis 3 bar Überdruck durchgeführt. Ganz besonders günstige Isomerisierungsparameter sind ~100 mbar bis Normaldruck (etwa 1 hPa) in Kombination mit der Siedetemperatur des α-Isophorons.

Das erfindungsgemäße Verfahren läßt sich in einer besonders bevorzugten Verfahrensweise kontinuierlich betreiben. In einer weiteren bevorzugten Ausführungsform trennt man Isomerisierung und Reindestillation voneinander.Die das Isomerisat enthaltende Flüssigphase wird nach deren Abtrennung zur Trennung von α-Isophoron und β-Isophoron unter Vakuum destilliert.

Die Destillation findet dann bei Temperaturen statt, bei denen die thermische bedingte Rückisomerisierung weitgehend ausgeschlossen ist.

Dabei erweist es sich als vorteilhaft, das Sumpfprodukt der Destillation in die Isomerisierungsstufe zu recyclieren.

Die Zufuhr von Frisch-α-Isophoron in die Isomerisierung wird so abgestimmt, daß das als β-IP abgetrennte Volumen ausgeglichen wird. Je nach β-IP Raum Zeit Ausbeute können so bis zu 10 vol% Frisch-α-IP der Isomerisierung zugeführt werden.

Der Volumenstrom an Recyclier-α-IP aus der Reindestillation kann je nach Bedingungen bis zu 90 vol% bezogen auf das ursprüngliche Volumen der Isomerisierungslösung betragen.

Am Kopf der Isomerisierungseinheit stellt sich je nach Verfahrensweise ein β-IP Gehalt von 1%-50% β-IP/α-IP ein. Dieses Gemisch wird anschließend einer Vakuumdestillation unterworfen, wobei ein β-IP-Produkt mit einer Reinheit > 97 % anfällt. Das Sumpfprodukt der Kolonne wird ohne weitere Reinigung auf die Isomerisierungseinheit zurückgefahren.

Auch hinsichtlich des Nebenproduktanfalls (im Vergleich z.b. mit D1, wo stöchiometrische Mengen Grignardverbindung verbraucht werden)und der auf das Volumen der Eduktlösung bezogene Raum-Zeit-Ausbeute ist das neue Verfahren dem Stand der Technik deutlich überlegen. Bei der Benutzung eines salzartigen oder metallorganischen Katalysators im Konzentrationsbereich $10^{-4}$ - 10 Mol% bezüglich im Isomerisierungsvolumen eingesetzten α-IP werden Selektivitäten > 99% erreicht, wodurch die Hochsiederentstehung im Vergleich zu im Stand der Technik beschriebenen Verfahren deutlich gesenkt werden kann.

Beispiel 1:

Es wird käufliches, technisches Natriumcarbonat als basischer Katalysator der Isomerisierung verwendet. Die Apparatur zur Durchführung der Isomerisierung besteht aus einem Heizpilz, einem Isomerisierungskolben mit aufgesetzter Destillationskolonne, über die ein βrimärgemisch einer Mischung aus α-IP und β-IP abgenommen wird, und einer Pumpe zur Recyclierung des nach β-IP Reindestiallation nicht umgesetzten α-Isophorons. Man legt 800 ml technisches α-Isophoron vor (Firma Atochem > 98%; = 723 g) und rührt eine den Tabellenwerten entsprechende Menge Natriumcarbonat ein. Man erhitzt die Suspension bei Normaldruck bis zur Siedetemperatur und stimmt die Menge des über eine Telabpumpe zugeförderten α-IP's und des abgenommenen Destillats aufeinander ab. Bei einem konstanten Abnahmevolumen von 25 vol% bezüglich eingesetztem α-IP, und einem konstanten Rücklaufverhältnis der Primärdestillation ergeben sich als Funktion verschiedener Katalystorkonzentrationen folgende Hochsiederbildungsraten, β-IP Bildungsraten und β-IP-Selektivitäten:

| Mol% $Na_2CO_3$ [Mol% pro Mol α-IP] | Bildungsrate HS* [g HS/h/l α-IP] | Bildungsrate β-IP [g β-TP/h/l α-IP | Selektivität β-IP |
|---|---|---|---|
| $2 \times 10^{-2}$ | 0,6 | 47,4 | 96,3 |
| $2 \times 10^{-1}$ | 0,6 | 63,5 | 99,1 |
| $2 \times 10^{0}$ | 1,7 | 72 | 97,7 |

*HS: Hochsieder = Sammelbegriff für Nebenprodukte der Isomerisierung

Die Sumpftemperatur der Isomerisierung bliebt während der Reaktionsdauer konstant. Das anfallende Primärdestillat wird auf eine Destillationskolonne gefordert, die bei einem Unterdruck von 5 mbar - 100 mbar arbeitet. Das bei 12 mbar anfallende Kopfprodukt hat einen Siedepunkt von 55 - 58 °C und besteht zu > 99% aus β-Isophoron. Bei der beschriebenen Dimensionierung des Versuchs entstehen pro Stunde und Liter α-IP Volumen im Isomerisierungsreaktor 47,4 -72 g β-Isophoron. Das als Sumpfprodukt nicht umgesetzte α-Isophoron wird auf den Isomerisierungsreaktor zurückgefahren. Die kalkulatorische Ausbeute bezogen auf die eingesetzte Katalysatormenge beträgt bei kontinuierlicher Fahrweise und bei 0,2 Mol% (= 0,2764 g Kat. /Mol IP) Katalysatorkonzentration $Y_{\beta\text{-IP}}$ = 229,7 kg β-IP/h/$kg_{Kat.}$.Die Raum-Zeit Ausbeute bezogen auf das Volumen der zu isomerisierenden Lösung beträgt $Y_{R\text{-}Z}$ = 0,0635 $l_{\beta\text{-IP}}$/h/$l_{Lösung}$.

Beispiel 2

Technisches Natriumhydroxid verschiedener Konzentrationen wird in die bereits beschriebene Apparatur in

vorgelegtes α-IP eingerührt. Bei gleicher kontinuierlicher Fahrweise (siehe Beispiel 1) wird am Kopf der Isomerisierungseinheit eine Abnahmegeschwindigkeit von 25 G% eines Primärdestillats bezogen auf die Isomerisierungsmenge über das Rücklaufverhältnis eingestellt. Man erhält nach HPLC Quantifizierung von Umsatz und Selektivität der β-IP Bildung folgende Ergebnisse:

| Mol% NaOH βMol% pro Mol α-IP] | Bildungsrate HS* [g HS/h/l α-IP] | Bildungsrate β-IP [g β-IP/h/l α-IP | Selektivität β-IP |
|---|---|---|---|
| $2 \times 10^{-2}$ | 0,5 | 54,6 | 99,1 |
| $2 \times 10^{-1}$ | 35,4 | 79,5 | 69,2 |

*HS: Hochsieder = Sammelbegriff für Nebenprodukte der Isomerisierung

Die Katalysatoraktivität bleibt während der Versuchsdauer unverändert.

Beispiel 3:

Technisches Kaliumcarbonat verschiedener Konzentrationen wird in die bereits beschriebene Apparatur in vorgelegtes α-IP eingerührt. Bei gleicher kontinuierlicher Fahrweise (siehe Beispiel 1) wird am Kopf der Isomerisierungseinheit eine Abnahmegeschwindigkeit von 25 G% eines Primärdestillats bezogen auf die Isomerisierungsmenge über das Rücklaufverhältnis eingestellt. Man erhält nach HPLC Quantifizierung von Umsatz und Selektivität der β-IP Bildung folgende Ergebnisse:

| Mol% $K_2CO_3$ [Mol% pro Mol α-IP] | Bildungsrate HS* [g HS/h/l α-IP] | Bildungsrate β-IP [g β-IP/h/l α-IP | Selektivität β-IP |
|---|---|---|---|
| $2 \times 10^{-2}$ | 0,9 | 73,8 | 98,9 |
| $2 \times 10^{-1}$ | 26,3 | 76,4 | 74,4 |
| $2 \times 10^{0}$ | 57,2 | 68,8 | 54,6 |

*HS: Hochsieder = Sammelbegriff für Nebenprodukte der Isomerisierung

Die Katalysatoraktivität bleibt während der Versuchsdauer unverändert.

Beispiel 4:

Man vergleicht verschiedene Alkalimetallkationen enthaltende Katalysatoren auf ihre katalytische Aktivität bei der Isomerisierung unter den in den vorgehneden Beispielen entsprechenden Bedingungen.

| Mol% Base [Mol% pro Mol α-IP] | Bildungsrate HS* [g HS/h/l α-IP] | Bildungsrate β-IP [g β-IP/h/l α-IP | Selektivität β-IP |
|---|---|---|---|
| $2 \times 10^{-2}$ LiOH | 3,0 | 61,3 | 95,3 |
| $2 \times 10^{-2}$ $Li_2CO_3$ | 0,4 | 48,3 | 99,2 |
| $2 \times 10^{-2}$ KOH | 3,9 | 66,9 | 94,5 |
| $2 \times 10^{-2}$ Na(OMe) | 0,7 | 46,9 | 98,5 |

*HS: Hochsieder = Sammelbegriff für Nebenprodukte der Isomerisierung

Beispiel 5:

Man vergleicht verschiedene Alkali- und Erdalkalibasen als Katalysatoren auf ihre katalytische Aktivität bei der Isomerisierung unter den in den vorgehenden Beispielen entsprechenden Bedingungen.

| Mol% Base [Mol% pro Mol α-IP] | Bildungsrate HS* [g HS/h/l α-IP] | Bildungsrate β-IP [g β-IP/h/l α-IP | Selektivität β-IP |
|---|---|---|---|
| 2 x $10^{-2}$ $Ca(OH)_2$ | 0,4 | 41,1 | 96,7 |
| 2 x $10^{-2}$ NaH | 0,8 | 58,6 | 96,6 |
| 2 x $10^{-2}$ $Mg(OH)_2$ | 0,8 | 8,2 | 91,5 |
| 2 x $10^{-1}$ $Mg(OH)_2$ | 0,7 | 9,3 | 93,3 |

*HS: Hochsieder = Sammelbegriff für Nebenprodukte der Isomerisierung

Beispiel 6:

Man setzt 1 Mol% Natriumcarbonat als basischen Katalysator der Isomerisierung unter vorgehend beschriebenen Bedingungen ein: Man gelangt zu einer Raum-Zeit Ausbeute pro Stunde und eingesetzter Katalysatormenge von Y = 15,357 kg ß-IP/h/kg Katalysator. Dies entspricht einer Raum-Zeit Ausbeute bezogen auf eingesetztem α-IP [l α-IP] von $Y_{R-Z}$ = 0,086 kg β-IP/h/l. Die per HPLC quantifizeirte Hochsiederbildungsrate beträgt 1,1 g HS/h/l. Das entspricht einer Selektivität der β-IP Bildung von 98,8%.

Beispiel 7:

Man vergleicht verschiedene Alkalimetallkationen enthaltende Katalysatoren auf ihre katalytische Aktivität bei der Isomerisierung unter den in den vorgehenden Beispielen entsprechenden Bedingungen.

| Mol% Base [Mol%/Mol α-IP] | $pK_a$* | Bildungsrate HS**[g HS/h/l α-IP] | Bildungsrate β-IP [g β-IP/h/l α-IP | Selektivität β-IP |
|---|---|---|---|---|
| 2 x $10^{-2}$ $NaNH_2$ | 36 | 1,0 | 49,6 | 97,9 |
| 2 x $10^{-2}$ $NaHCO_3$ | 6,4 | 0,8 | 55,1 | 98,6 |
| 2 x $10^{-2}$ $KHCO_3$ | 6,4 | 3,0 | 64,4 | 95,5 |
| 2 x $10^{-2}$ $Na(CH_3CO_2)$ | 4,8 | 0,7 | 49,2 | 98,7 |

* pka-Angaben beziehen sich auf die Basizität des Anions im Gleichgewicht mit der korrespondierenden protonierten Stufe

**HS: Hochsieder = Sammelbegriff für Nebenprodukte der Isomerisierung

Beispiel 8:

Man vergleicht verschiedene Alkalimetallkationen enthaltende Katalysatoren auf ihre katalytische Aktivität bei der Isomerisierung unter den in den vorgehenden Beispielen entsprechenden Bedingungen.

| Mol% Base [Mol%/Mol α-IP] | $pK_a$* | Bildungsrate HS**[g HS/h/l α-IP] | Bildungsrate β-IP [g β-IP/h/l α-IP | Selektivität β-IP |
|---|---|---|---|---|
| kein Kat. | / | 0 | 8,0 | 100 |
| 2 x $10^{-2}$ $KHSO_4$ | - 5,2 | 0,7 | 44,7 | 98,5 |

* pka-Angaben beziehen sich auf die Basizität des Anions im Gleichgewicht mit der korrespondierenden protonierten Stufe
**HS: Hochsieder = Sammelbegriff für Nebenprodukte der Isomerisierung

Beispiel 9

Man vergleicht verschiedene Alkalimetallkationen enthaltende Katalysatoren und je Elemente auf ihre katalytische Aktivität bei der Isomerisierung unter den in den vorgehenden Beispielen entsprechenden Bedingungen.

| Mol% $Na_2CO_3$ [Mol% pro Mol α-IP] | Bildungsrate HS* [g HS/h/l α-IP] | Bildungsrate β-IP [g β-IP/h/l α-IP | Selektivität β-IP |
|---|---|---|---|
| 2 x $10^{-2}$ $LiAlH_4$ | 0,8 | 43,3 | 98,3 |
| 2 x $10^{-2}$ $LiBH_4$ | 0,8 | 46,1 | 98,3 |
| 2 x $10^{-2}$ Na | 0,7 | 53,7 | 98,7 |

* pka-Angaben beziehen sich auf die Basizität des Anions im Gleichgewicht mit der korrespondierenden protonierten Stufe

Vergleichsbeispiel 1:

Verfährt man wie in der japanischen Offenlegungsschrift (A) HEI 1-175954 beschrieben (300 g α-IP; 25,7g $SiO_2$, Abnahmegeschwindigkeit 5g/h) und zieht ein 89% β-IP/α-IP Gemisch/h unter Benutzung von $SiO_2$ als Katalysator ab, so beträgt die auf die Katalysatormenge bezogene Ausbeute Y=0,174 $kg_{\beta\text{-IP}}/h/kg_{Kat.}$.Die Raum-Zeit Ausbeute bezogen auf die eingesetzte Eduktlösung beträgt $Y_{R\text{-}Z}$ = 0,0149 $l_{\beta\text{-IP}}/h/l_{Lösung}$.

**Patentansprüche**

1. Verfahren zur Herstellung von 3,5,5-Trimethylcyclohexa-3-en-1-on (β-Isophoron) durch Isomerisierung von 3,5,5-Trimethylcyclohexa-2-en-1-on (α-Isophoron) in Gegenwart eines Katalysators,ohne Zusatz einer weiteren, organischen Base, bei 100 bis 260 ° C in der flüssigen Phase
**dadurch gekennzeichnet,**
daß man als Katalysator eine salzartige Verbindung eines Elements der Gruppen $I_a$ oder $II_a$ oder die Elemente der Gruppen $I_a$ und $II_a$ selbst verwendet.

2. Verfahren nach Anspruch 1,
**dadurch gekennzeichnet,**
daß man als Katalysator Alkalioxide, Alkali- und Erdalkaliverbindungen des Typs Hydroxid, Carbonat, Hydrogencarbonat, Hydrid, komplexes Hydrid, Amid, Alkoholat, Mercaptid, Carboxylat, Bromid, Fluorid, Cyanid, Hydrogensulfid,Hydrogensulfat, Sulfid, oder metallorganische Alkali und Erdalkaliverbindungen, oder gegebenfalls deren Mischungen einsetzt.

3. Verfahren nach Anspruch 2,
**dadurch gekennzeichnet,**
daß man ein Alkali- oder Erdalkall- Carbonat oder - Hydroxid verwendet.

4. Verfahren nach Anspruch 3,
**dadurch gekennzeichnet,**
daß man ein Hydroxid oder Carbonat der Elemente Natrium oder Kalium einsetzt.

5. Verfahren nach einem der vorhergehenden Ansprüche,
**dadurch gekennzeichnet,**
daß man den Katalysator in einem Verhältnis von zwischen $10^{-4}$ und 5 Mol-% bezogen auf $\alpha$-Isophoron einsetzt.

6. Verfahren nach Anspruch 5,
**dadurch gekennzeichnet,**
daß man den Katalysator in einem Verhältnis von zwischen $10^{-3}$ und 5 Mol% bezogen auf $\alpha$-Isophoron einsetzt.

7. Verfahren nach Anspruch 6,
**dadurch gekennzeichnet,**
daß man den Katalysator in einem Verhältnis von zwischen 0,01 und 2 Mol% bezogen auf $\alpha$-Isophoron einsetzt.

8. Verfahren nach einem oder mehreren der vorhergehenden Ansprüche,
**dadurch gekennzeichnet,**
daß man bei Normaldruck (etw. 1 hPa) und der Siedetemperatur des $\alpha$-Isophorons isomerisiert.

9. Verfahren nach einem oder mehreren der vorhergehenden Ansprüche ,
**dadurch gekennzeichnet,**
daß das Verfahren kontinuierlich betrieben wird.

10. Verfahren nach Anspruch 9,
**dadurch gekennzeichnet,**
daß man das Sumpfprodukt der Destillation, das den aktiven Katalysator und nicht umgesetztes $\alpha$-Isophoron enthält, in die Isomerisierung recycliert.

11. Verfahren nach einem oder mehreren der vorhergehenden Ansprüche,
**dadurch gekennzeichnet,**
daß man die Isomerisierung bei einer Temperatur von 150 bis 250 °C und einem entsprechenden die Flüssigphase aufrechterhaltenden Druck durchführt, kontinuierlich Reaktionsgemisch abzieht und dieses bei einem Druck von 100 bis $3 \times 10^4$ Pa destilliert und den Destillationssumpf gegebenenfalls in die Isomerisierung zurückführt.

Europäisches Patentamt

# EUROPÄISCHER RECHERCHENBERICHT

Nummer der Anmeldung
EP 99 10 8497

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 832 870 A (DEGUSSA)<br>1. April 1998 (1998-04-01)<br>* das ganze Dokument * | 1-3,5-11 | C07C45/67<br>C07C45/82<br>C07C49/603 |
| X | FR 2 253 730 A (FIRMENICH & CIE)<br>4. Juli 1975 (1975-07-04)<br>* Seite 5; Anspruch 9 * | 1-4,11 | |
| Y | US 3 397 120 A (DIANA WILLIAM D ET AL)<br>13. August 1968 (1968-08-13)<br>* Spalte 7, Zeile 66 - Zeile 72; Anspruch 1 * | 1-4 | |
| Y | US 2 246 032 A (BENT FRANKLIN A)<br>17. Juni 1941 (1941-06-17)<br>* Seite 3, Spalte 1, Zeile 37 - Zeile 38; Ansprüche * | 1-4 | |

RECHERCHIERTE SACHGEBIETE (Int.Cl.6)
C07C

Der vorliegende Recherchenbericht wurde für alle Patentansprüche erstellt

| Recherchenort | Abschlußdatum der Recherche | Prüfer |
|---|---|---|
| DEN HAAG | 21. Juli 1999 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 99 10 8497

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-07-1999

| Im Recherchenbericht angeführtes Patentdokument | Datum der Veröffentlichung | Mitglied(er) der Patentfamilie | Datum der Veröffentlichung |
|---|---|---|---|
| EP 0832870 A | 01-04-1998 | DE 19639570 A | 02-04-1998 |
| | | JP 10168023 A | 23-06-1998 |
| FR 2253730 A | 04-07-1975 | CH 585168 A | 28-02-1977 |
| | | DE 2457157 A | 12-06-1975 |
| | | GB 1429120 A | 24-03-1976 |
| | | JP 948890 C | 20-04-1979 |
| | | JP 50093946 A | 26-07-1975 |
| | | JP 53028907 B | 17-08-1978 |
| | | NL 7415846 A | 10-06-1975 |
| | | US 4010205 A | 01-03-1977 |
| | | US 4026948 A | 31-05-1977 |
| US 3397120 A | 13-08-1968 | DE 1643962 A | 15-07-1971 |
| | | FR 1553211 A | 10-01-1969 |
| | | GB 1168009 A | 22-10-1969 |
| US 2246032 A | 17-06-1941 | US 2197462 A | 16-04-1940 |

EPO FORM P0461

Für nähere Einzelheiten zu diesem Anhang : siehe Amtsblatt des Europäischen Patentamts, Nr.12/82